# EUROPEAN PATENT APPLICATION

(11) **EP 1 477 195 A1**
(43) Date of publication of application: **17.11.2004**
(21) Application number: 02805902.0
(22) Date of filing: 26.12.2002
(51) Int. Cl.: A61M 5/24

(54) **CARTRIDGE SYRINGE, CARTRIDGE AND OPHTHALMOLOGIC SYRINGE SET HAVING SUCH SYRINGE**

(30) Priority: 27.12.2001 JP 2001395911; 02.08.2002 JP 2002225981; 02.08.2002 JP 2002225982
(71) Applicant: Showa Yakuhin Kako Co., Ltd, Tokyo 104-0031 (JP); MENICON CO., LTD., Nagoya-shi, Aichi 460-0006 (JP)
(72) Inventor: KAWASAKI, Yoshihiko, SHOWA YAKUHIN KAKO CO., LTD., Tokyo 104-0031 (JP); SHIMODA, Yoichi, c/o SHOWA YAKUHIN KAKO CO., LTD., Tokyo 104-0031 (JP); SHISHIDO, Yoshihiro, Tsuzuki-ku, Yokohama-shi, Kanagawa 224-0 (JP); ENDO, Takahiro, c/o MENICON CO., LTD., Nagoya-shi, Aichi 460-0006 (JP); KONDO, Naoki, c/o MENICON CO., LTD., Nagoya-shi, Aichi 460-0006 (JP); TAKESHITA, Kenji, c/o MENICON CO., LTD., Nagoya-shi, Aichi 460-0006 (JP)
(74) Representative: Sternagel, Fleischer, Godemeyer & Partner
(86) International application number: PCT/JP2002/013628
(87) International publication number: WO 2003/055546

(57) **Abstract**

An object of the present invention to provide a cartridge syringe and an ophthalmologic syringe set equipped with the cartridge syringe, where the cartridge syringe can be used repeatedly by only exchanging the cartridge containing an ophthalmologic therapeutic agent, and to reduce the amount of waste products and ensure storage space for the ophthalmologic therapeutic agents. The cartridge syringe 1 of the present invention comprises a syringe body 3 having a cartridge housing portion 11, a needle holding member 5 that is disposed in a connectable manner to the tip of the syringe body 3, and a plunger 7 that is slidably disposed on a base-end side of the syringe body 3, the plunger 7 having a pusher 37 at the tip thereof, wherein a one-touch loading mechanism is provided in the syringe body 3 or at a coupling portion between the syringe body 3 and the plunger 7, the one-touch loading mechanism allowing the position of the plunger 7 to be temporarily changed such that the cartridge 9 can be loaded into the syringe body 3.

## Description

### FIELD OF THE INVENTION

The present invention relates to a cartridge syringe used for administering ophthalmologic therapeutic agent, a cartridge, and an ophthalmologic syringe set equipped with the cartridge syringe.

### BACKGROUND ART

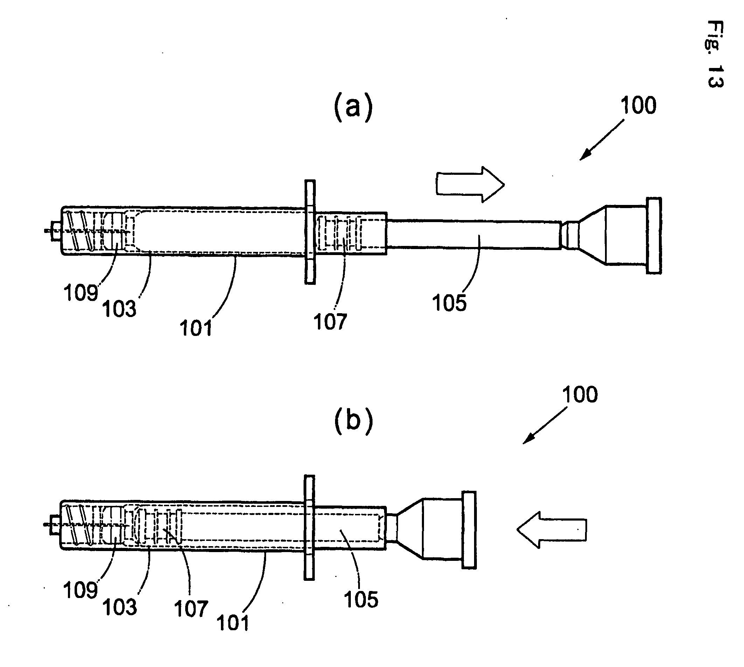

In a conventional ophthalmologic syringe as shown in Fig. 13, a drug solution container 103 containing an ophthalmologic therapeutic agent is housed in a syringe body 101. A plunger 105 is connected to a base-end of the syringe body 101. At the tip of the plunger 105, there is formed a piston 107, and the drug solution container 103 is sealed on its base-end side by the piston 107. A rubber stopper 109 is mounted at the tip of the drug solution container 103 in a manner such that a needle can be stuck into the rubber stopper 109 when the ophthalmologic syringe 100 is used.

Thus, the conventional ophthalmologic syringe, which is disposable, is made up of the syringe body 101, drug solution container 103, and plunger 105 as integral components. The ophthalmologic syringe, therefore, must be disposed of as a whole after use, resulting in large amounts of waste products.

The ophthalmologic syringes are individually contained in hermetically sealed blister packages to prevent contamination by microbes or the like. The blister packages are further contained in outer packages to protect the blister packages and/or syringes. The blister packages are made of plastic, and a relatively small example thereof measures approximately 16 cm in width, 4.5 cm in length, and 3 cm in height. The outer packages are made of paper and are larger. A relatively small outer package measures approximately 16.5 cm in width, 5 cm in length, and 3.5 cm in height.

Thus, the syringes in these packages are very bulky and require significant storage space if a large number of them are to be kept in stock. Further, an ophthalmologic syringe after use, its blister package, and the outer package together weigh about 28.5 g. The presence of packaging materials is also a cause of an increase in waste products.

The aforementioned problem of storage space is strongly felt with regard to ophthalmologic therapeutic agents, which are often refrigerated during storage due to their tendency to easily decompose due to heat or light. For example, in the case of an ophthalmologic syringe in which an aqueous solution containing sodium hyaluronate of high molecular weight as an active ingredient is employed as an ophthalmologic therapeutic agent, it is preferable to refrigerate the syringe as the viscosity of the therapeutic agent decreases at high storage temperatures. However, a large storage space in the drug storage refrigerator is required if an outer package is large.

In particular, with regard to syringes that are filled with viscoelastic substances which are used for cataract surgeries, as many as 15 cataract surgeries may be performed on a daily basis at hospitals with many surgical cases, reflecting the development of cataract surgical technologies. At such hospitals, as many as 15 to 30 of the bulky, outer packages containing the syringes must be kept in stock at all times, causing a serious storage space problem in drug storage refrigerators.

The syringes for ophthalmologic therapeutic agents are more often used for injecting a drug into tissue that has been incised, than for suction purposes as in blood sampling. Therefore, from the viewpoint of using the syringe more than once, it is in many cases more useful to allow the plunger to be easily detached from the piston stopper after the injection of drug, rather than to put an emphasis on the integral nature of the piston stopper and plunger in the cartridge.

### DISCLOSURE OF THE INVENTION

In view of the background of the invention mentioned in the aforementioned prior art, it is an object of the present invention to provide a cartridge syringe, a cartridge, and an ophthalmologic syringe set equipped with the cartridge syringe, where the cartridge syringe is adapted such that the cartridge syringe can be used repeatedly by exchanging and discarding only the cartridge containing an ophthalmologic therapeutic agent after use. By providing these, the invention aims to reduce the amount of waste products and ensure storage space for the ophthalmologic therapeutic agents. Another object of the present invention is to enable the plunger to be easily separated from the piston stopper so that the cartridge can be easily replaced.

Thus, the present invention provides a cartridge syringe for use with a flanged cartridge that is filled with an ophthalmologic therapeutic agent, wherein the cartridge syringe comprises a syringe body having a cartridge housing portion, a needle holding member that is disposed in a connectable manner to the tip of the syringe body, and a plunger that is slidably disposed on a base-end side of the syringe body, the plunger having a pusher at the tip thereof, wherein a one-touch loading mechanism is provided in the syringe body or at a coupling portion between the syringe body and the plunger, the one-touch loading mechanism allowing the position of the plunger to be temporarily changed such that the cartridge can be loaded into the syringe body.

In the aforementioned cartridge syringe, the one-touch loading mechanism may comprise a knuckle by which the syringe body and the plunger are coupled in a foldable manner, whereby the cartridge can be loaded by folding the plunger.

In the aforementioned cartridge syringe, a cartridge-holding portion may be mounted around the plunger slidably with respect to the syringe body, the cartridge-holding portion being biased towards the tip of the cartridge syringe such that when the cartridge is loaded in the cartridge housing portion, the cartridge can be held in place in such a way that the base-end side of the cartridge is pushed by the cartridge-holding portion.

A cam face may be formed at the tip of the knuckle such that the cam face can slide on a cam slide face formed at the base-end side of the syringe body, thereby allowing the knuckle to be turned. In this structure, when the plunger is fully pulled and then folded towards the side on which the cam face is formed, the cam face slides on the cam slide face, thereby allowing the folding of the plunger.

The plunger may be adapted to be foldable in only a single direction.

A flange support portion may be formed on the base-end side of the syringe body such that, when the cartridge is loaded into the cartridge-housing portion, the flange portion of the cartridge abuts the flange support portion, thereby limiting the insertion of the cartridge.

Two locking claws may be formed on the base-end side of the syringe body, the locking claws opposed to each other and extending in the longitudinal direction. In this structure, the loading of the cartridge is completed when two locking faces formed on the flange portion of the cartridge for regulating the orientation of the cartridge are positioned such that they are opposite the insides of the locking claws. When the loading of the cartridge is thus completed, the locking faces on the cartridge engage the inside of the locking claws, so that the free rotation of the cartridge around the longitudinal axis of the cartridge may be limited.

When the cartridge is loaded in the cartridge housing portion, as the plunger is turned to be linearly oriented with respect to the syringe body, the cartridge-holding portion may be biased towards the syringe body such that the flange portion of the cartridge may be retained between the cartridge-holding portion and the flange support portion.

In the aforementioned cartridge syringe, the one-touch loading mechanism may be structured such that it can be dismantled by slightly turning the plunger holder holding the plunger around the longitudinal axis of the syringe body.

In the aforementioned cartridge syringe, the one-touch loading mechanism may be structured such that the plunger holder holding the plunger may be slid in a direction perpendicular to the longitudinal axis of the syringe body.

In the aforementioned ophthalmologic syringe set, an integral coupling structure may be provided between a pusher provided on the tip of the plunger and a piston stopper provided on the base-end side of the cartridge, whereby the pusher and the piston stopper are coupled in an integral manner.

In the aforementioned ophthalmologic syringe set, the integral coupling structure may comprise a push-in portion provided on the pusher and an insertion hole formed in the piston stopper.

In the aforementioned cartridge syringe, a tapered push-in portion may be formed more towards the tip side than the pusher, the push-in portion being adapted to be inserted into an insertion hole formed on the base-end side of the piston stopper of the cartridge.

The integral coupling structure may comprise an externally threaded convex portion formed on the pusher and an internally threaded concave portion formed on the piston stopper.

The present invention also relates to a cartridge filled with an ophthalmologic therapeutic agent for use in combination with the aforementioned cartridge syringe of the invention.

The ophthalmologic syringe set of the invention comprises any of the cartridge syringes described above, and a cartridge filled with an ophthalmologic therapeutic agent.

In the aforementioned ophthalmologic syringe set, the ophthalmologic therapeutic agent with which the cartridge is filled may be a viscoelastic substance.

In the aforementioned ophthalmologic syringe set, the ophthalmologic therapeutic agent with which the cartridge is filled may be an aqueous solution containing hyaluronic acid or its salts as an active ingredient.

In the aforementioned ophthalmologic syringe set, the ophthalmologic therapeutic agent with which the cartridge is filled may be an aqueous solution containing sodium hyaluronate as an active ingredient.

In the aforementioned ophthalmologic syringe set, the volume of the cartridge may be from 1.0 mL to 20.0 mL.

### BRIEF DESCRIPTION OF THE DRAWINGS

Fig. 1 is an exploded perspective view of a first example of the cartridge syringe of the present invention and the ophthalmologic syringe set equipped with the cartridge syringe.
Fig. 2 is an exploded perspective view of a second example of the cartridge syringe of the present invention and the ophthalmologic syringe set equipped with the cartridge syringe.
Fig. 3 is a side view illustrating how the cartridge is loaded in the first example of the cartridge syringe according to the present invention.
Fig. 4 is a side view illustrating how the cartridge is loaded in the second example of the cartridge syringe according to the present invention.
Fig. 5 shows a first example of the ophthalmologic syringe set according to the present invention. Fig. 5(a) is a plan view of the ophthalmologic syringe set after the cartridge has been loaded. Fig. 5(b) is a plan view of the ophthalmologic syringe set as seen from the lengthwise direction of the cartridge syringe when the cartridge is loaded.
Fig. 6 shows a second example of the ophthalmologic syringe set according to the present invention. Fig. 6(a) is a plan view of the ophthalmologic syringe set after the cartridge has been loaded. Fig. 6(b) is a plan view of the ophthalmologic syringe set as seen from the lengthwise direction of the syringe when the cartridge is loaded.
Fig. 7(a) is a perspective view illustrating how a flange of the cartridge is guided by guide edges, immediately before the flange is automatically slid into a space between two locking claws. Fig. 7(b) is a perspective view showing the locking face of the flange of the cartridge which has been placed between the locking claws.
Fig. 8 is an exploded perspective view of a first example of another embodiment of the present invention.
Fig. 9 is an exploded perspective view of a second example of another embodiment of the present invention.
Fig. 10 shows plan views of two embodiments of the one-touch loading mechanism in the first example of the ophthalmologic syringe set according to the present invention.
Fig. 11 shows plan views of two embodiments of the one-touch loading mechanism in the second example of the ophthalmologic syringe set according to the present invention.
Fig. 12 is a cross section of another embodiment of the integral coupling structure in the first example of the ophthalmologic syringe set according to the present invention.
Fig. 13(a) is a side view of a disposable syringe according to the prior art, before use. Fig. 13(b) is a side view of the disposable syringe according to the prior art, after use.

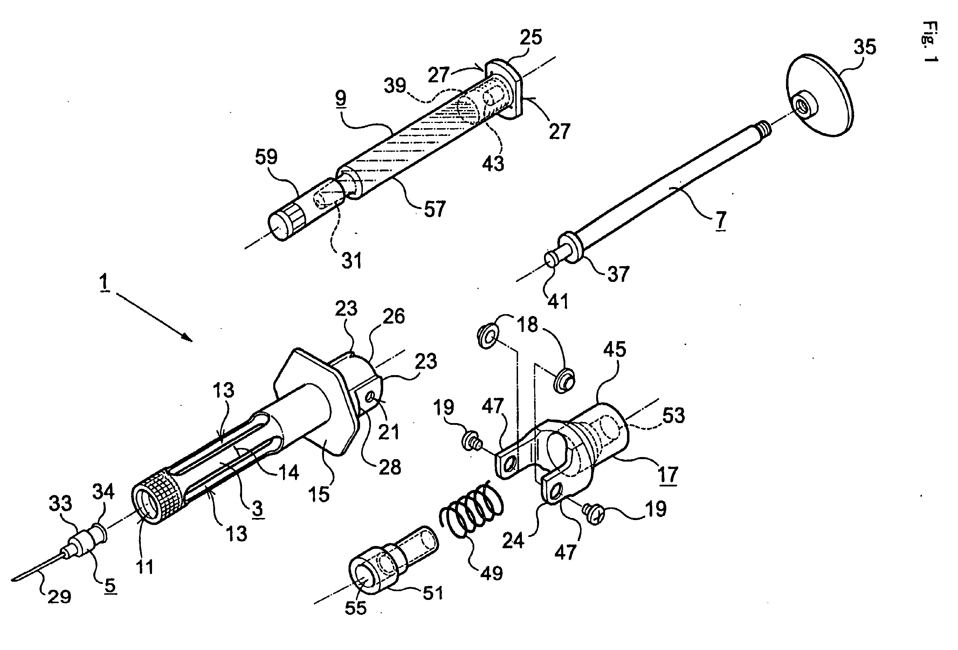

In Figs. 1 to 13: 1, cartridge syringe; 3, syringe body; 5, needle-holding member; 7, plunger; 9, cartridge; 11, cartridge-housing portion; 13, remaining-amount monitoring window; 14, calibration line; 15, gripping portion; 17, knuckle; 18, washer; 19, securing screw; 21, screw hole; 23, locking claw; 24, cam face; 25, flange portion; 26, flange support portion; 27, locking face; 28, cam slide face; 29, needle; 31, small-diameter portion; 33, socket portion; 34, flange portion; 35, handle; 37, pusher; 39, piston stopper; 41, push-in portion; 43, insertion hole; 45, holder portion; 47, rotary blade; 48, guide edge; 49, compression coil spring; 51, cartridge-holding portion; 53, fitting hole; 55, pusher receiving hole; 57, cartridge body; 59, rubber stopper; 61, receiving opening portion; 63, locking claw; 65, locking groove; 67, plunger holder; 69, guide rail; 71, plunger holder; 73, slider; 75, threaded convex portion; 77, threaded concave portion; 100, ophthalmologic syringe; 101, syringe body; 103, drug solution container; 105, plunger; 107, piston; and 109, rubber stopper.

### BEST MODE OF CARRYING OUT THE INVENTION

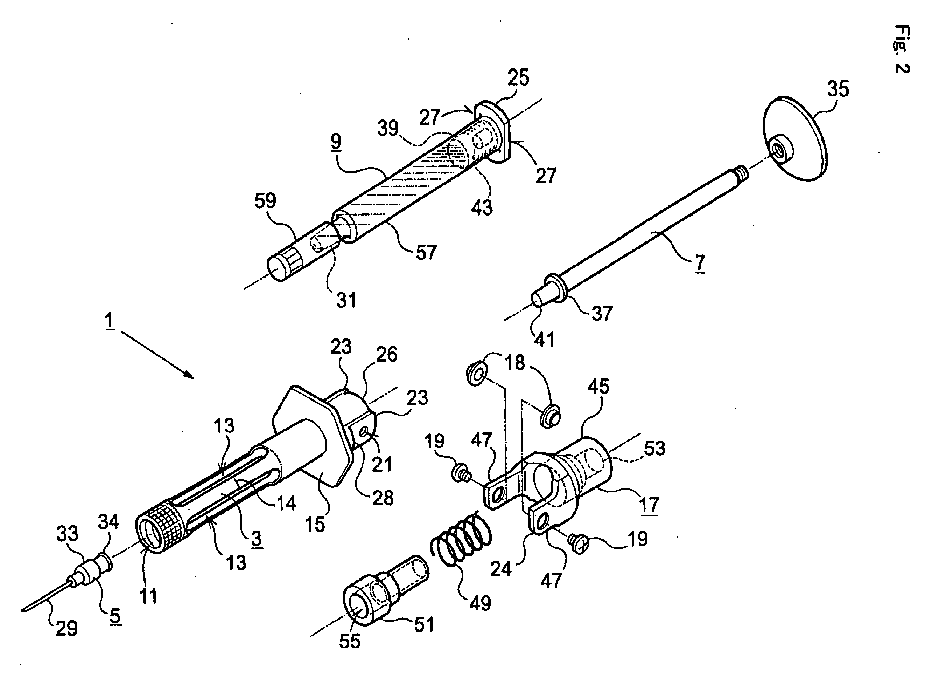

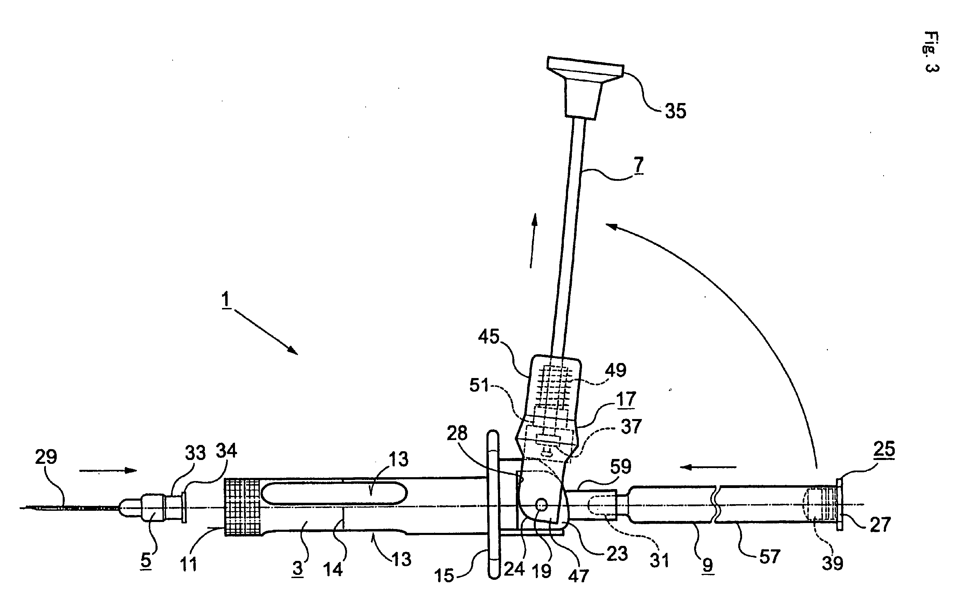

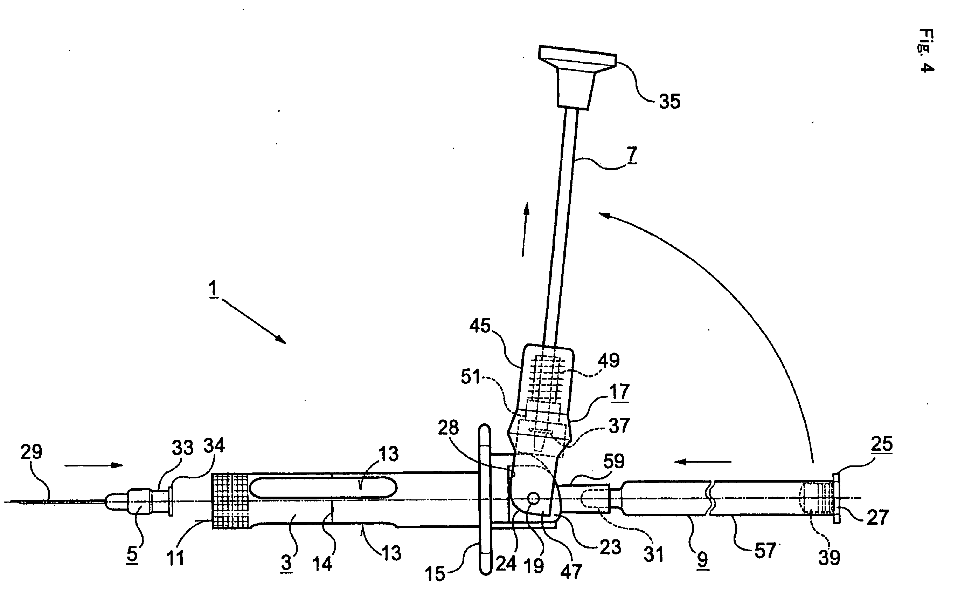

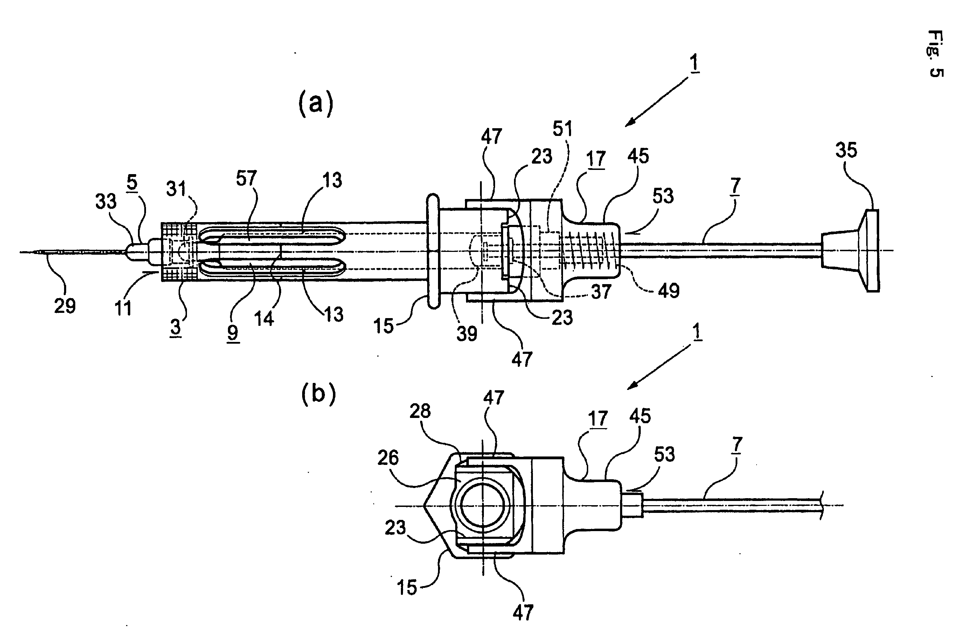

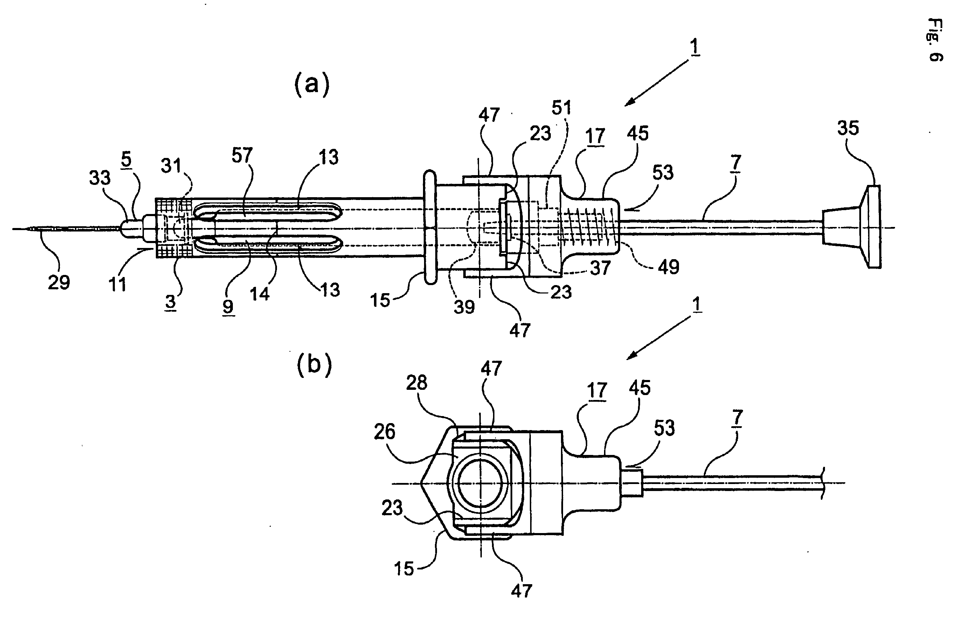

Embodiments of the invention will be described by referring to the drawings. Figs. 1 and 2 are exploded perspective views of the cartridge syringe of the present invention and an ophthalmologic syringe set equipped with the cartridge syringe. Figs. 3 and 4 are side views of the ophthalmologic syringe set when loading the cartridge. Figs. 5(a) and 6(a) are plan views of the ophthalmologic syringe set after the cartridge has been loaded. Figs. 5(b) and 6(b) are plan views of the ophthalmologic syringe set as seen in a longitudinal direction of the cartridge syringe when the cartridge is loaded.

Figs. 1, 3, and 5 show a first example of the cartridge syringe of the present invention and the ophthalmologic syringe set equipped with the cartridge syringe. Figs. 2, 4, and 6 show a second example of the cartridge syringe of the present invention and the ophthalmologic syringe set equipped with the cartridge syringe. In the second example, a push-in portion that tapers towards the tip is formed forwardly of the pusher. So that, the push-in portion is adapted to be inserted into an insertion hole formed in the base-end of a piston stopper of the cartridge.

A cartridge syringe 1 is basically made up of a syringe body 3, a needle holding member 5 mounted at the tip of the syringe body 3, and a plunger 7 mounted at the base-end of the syringe body 3. In the present specification, the tip refers to the end of the syringe body at which the needle of the cartridge syringe 1 is mounted, and the base-end refers to the opposite end of the syringe body.

The syringe body 3 is made of a cylindrical member in which a cartridge-housing portion 11 with a slightly larger inner diameter than the external diameter of the cartridge 9 is formed. On the peripheral surface of the syringe body 3, there are provided remaining-amount monitoring windows 13. In the vicinity of the windows 13, a calibration line 14 is provided to know the approximate remaining amount of the ophthalmologic therapeutic agent. The remaining-amount monitoring windows 13 are provided so as to monitor the remaining amount of the ophthalmologic therapeutic agent with which the cartridge 9 is filled, and the shape and/or the number thereof may be variously selected. Preferably, the number of the remaining-amount monitoring windows should be from 1 to 4 in light of the strength and visibility of the syringe body. In the embodiment shown in Figs. 1 to 6, three elliptical remaining-amount monitoring windows 13 are provided on opposed surfaces.

At the base-end of the syringe body 3, there is provided a flange-shaped gripping portion 15. The gripping portion 15 is adapted such that, when the cartridge syringe 1 is used, the index finger and middle finger can be placed on the gripping portion 15, thereby allowing the syringe body 3 to be held and facilitating the insertion of the plunger 7, as will be described later. Thus, a variety of shapes may be selected for the gripping portion 15 as long as they allow for the easy placement of the fingers. Preferably, the shape should be polygonal when seen in a plan view, such as a triangular shape. In the embodiment shown in Figs. 1 to 6, the gripping portion 15 is hexagonal when seen in a plan view, in light of the stability of the syringe body 3 when placed on a desk. In this embodiment, further, the cartridge syringe is formed to be heavier towards the plunger 7 as a fulcrum with respect to the gripping portion 15. Thus, the tip of the needle which is inserted into the eye can be prevented from coming into contact with the surface of the desk, the bottom of a sterilization tray, or other items. The attachment of germs or foreign matter to the tip of the needle can therefore be prevented, such that the risk of postoperative inflammations (such as infections) can be reduced. Further, the flange-shaped gripping portion 15 eliminates the need for the mount (the wall portion of the sterilization tray, tweezers, etc.) used in the conventional ophthalmologic syringes of the above-described type for preventing the needle tip from coming into contact with the surface of the desk or the bottom of the sterilization tray and so on.

The portion of the syringe body further towards the base-end beyond the gripping portion 15 forms a coupling portion with the plunger 7. The coupling portion is provided with a one-touch loading mechanism adapted to allow the cartridge 9 to be loaded into the syringe body 3 by a simple operation. In the embodiment shown in Figs. 1 to 6, the one-touch loading mechanism is adapted to couple the syringe body 3 with the plunger 7 by means of a knuckle 17. Two screw holes 21 are provided in the coupling portion into which the securing screws 19 which function as the rotation axles for the knuckle 17 can be mounted.

On the end face of the base-end of the syringe body 3, two locking claws 23 are provided opposite each other, extending in the longitudinal direction. Between the locking claws 23, there is formed a flange support portion 26 at a position lower than the location where the locking claws 23 are disposed. The locking claws 23 are adapted to be engaged with two, opposed locking faces 27 formed by cutting either side of a flange portion 25 at the base-end of the cartridge 9 in a straight line, so that the rotation of the cartridge 9 around its longitudinal axis can be prevented. Further, since the flange portion 25 of the cartridge comes into contact with the flange support portion 26 at the time of insertion of the cartridge, the flange support portion 26 has a function of limiting further insertion of the cartridge.

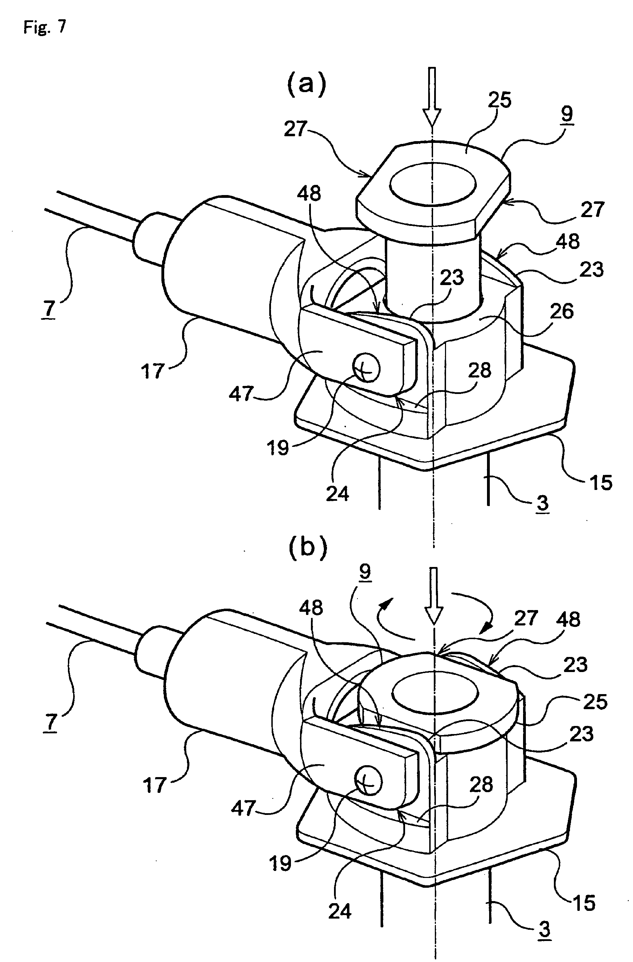

Alternatively, the locking claws 23 may be formed with diagonal guide edges 48 so as to allow the flange portion 25 with the locking faces 27 to be naturally guided between the two locking claws 23, as shown in Fig. 7(a) and (b). In this way, after the flange portion 25 of the cartridge 9 comes into contact with the guide edges 48, the cartridge 9 automatically rotates in the direction of inclination due to the weight of the cartridge 9, as shown in Fig. 7(b), whereby the flange portion 25 settles between the two locking claws 23 naturally. Instead of forming the locking claws 23 diagonally, the cartridge 9 may be provided with a taper.

The needle-holding member 5 comprises a needle 29 for direct administration of an ophthalmologic therapeutic agent, and a socket portion 33, which holds the needle 29 and is adapted to fit into a small-diameter portion 31 at the tip of the cartridge 9. At the base-end of the socket portion 33, there is formed a flange portion 34 which is adapted to be threaded into a internal thread formed on the internal peripheral surface of the tip of the syringe body 3. Alternatively, the socket portion 33 may be adapted to be press-inserted into the inside of the tip of the syringe body 3 and/or the small-diameter portion 31 at the tip of the cartridge 9.

The plunger 7 is made of a round-bar shaped axle formed with a handle 35 at the base-end thereof and a pusher 37 at the tip end thereof. When the cartridge syringe 1 is used, the user places his or her thumb, for example, on the handle 35 and holds the cartridge syringe 1 between the handle 35 and the gripping portion 15, so that he or she can push the pusher 37. Thus, the shape of the handle 35 may be varied as long as it is suitable for the pushing of the pusher 37. In the embodiment shown in Figs. 1 to 6, the handle 35 is circular when seen in a plan view with the center somewhat depressed such that the pusher 37 can be easily pushed with the thumb.

The pusher 37 is adapted to come into contact with the piston stopper 39, which is initially located towards the base-end of the cartridge 9, and directly press the piston stopper 39 towards the tip of the cartridge. In the embodiment shown in Figs. 1 to 6, the pusher 37 is formed in the shape of a disc with a diameter larger than the diameter of the axle. On the end face of the tip of the pusher 37, the push-in portion 41 is provided. In Figs. 2, 4 and 6, the push-in portion 41 which is tapered such that its diameter decreases towards the tip, is provided. The push-in portion 41 is inserted into an insertion hole 43 formed in the end face of the base-end of the piston stopper 39, and thereby the piston stopper 39 and the pusher 37 are integrally coupled, while allowing the piston stopper 39 to be moved. This type of mechanism for integrally coupling the piston stopper 39 and the pusher 37 will be defined as an integral coupling mechanism in the present specification.

In the second example of the ophthalmologic syringe set according to the present invention shown in Figs. 2, 4 and 6, the push-in portion 41 is formed in the shape of a conic trapezoid with a decreasing diameter. Therefore, when the piston stopper 39 is pushed towards the tip side by the pushing action of plunger 7, the push-in portion 41 can be firmly fitted in the insertion hole 43. Conversely, when the plunger 7 is pulled, the push-in portion 41 can be easily pulled out of the insertion hole 43, thereby preventing the piston stopper 39 from being moved towards the base-end side together with the plunger 7. In the case of ophthalmologic therapeutic agents, for which the present invention is adapted, a drug is more often ejected outside by the pushing of the plunger 7 than a liquid is sucked, as in the case of taking a blood sample. For such purposes, the above-described structure is advantageous more often than not. Further, this structure makes it possible to avoid the problem of the piston stopper 39 moving to the base-end of the cartridge 9 as the plunger 7 is pulled and remaining attached to the piston stopper 39, thereby preventing the folding of the plunger 7. Accordingly, the above-described structure allows the cartridge to be easily replaced. Further, by adopting this structure, the development of air bubbles in the cartridge due to the backward movement of the piston stopper 39 can be prevented. This makes it possible to eliminate the problem of the surgical field of view being narrowed by the air bubbles that flow into the eye along with the drug solution, or the need for complicated operations for eliminating air bubbles in the anterior chamber after surgery. It is particularly difficult to remove air bubbles once they develop in the cartridge in the case of ophthalmologic therapeutic agents such as hyaluronic acid or salts thereof, due to their high viscosity.

As mentioned above, the one-touch loading mechanism is provided in the connecting portion between the syringe body 3 and the plunger 7. In the embodiment shown in Figs. 1 to 6, the mechanism which is composed of the knuckle 17, by which the syringe body 3 and the plunger 7 can be coupled in a foldable manner, is adopted. The knuckle 17 is made up of a cylindrical holder portion 45, and two rotary blades 47 extending from the tip of the holder portion 45 like tongues. The securing screws 19 are entered from the outside of the rotary blades 47, and are put through convex washers 18, which are disposed on the inside of the rotary blades 47 with their bulges facing outward. The screws 19 are then threaded with the screw holes 21, such that the knuckle 17 can be rotated relative to the syringe body 3. The washers 18 allow the rotary blades 47 to be rotated smoothly and reduce the amount of frictional heat which is generated by the rotation of the rotary blades 47, thus preventing the fusion bonding of individual members.

On one side of the tip of each rotary blade 47, there is formed a circular-arc cam face 24, which is adapted to slide on a cam slide face 28 formed on the base-end side of the syringe body 3, thereby allowing the knuckle 17, namely the plunger 7, to be turned. The cam face 24 is not formed on the other side of the tip of each rotary blade 47, so that the plunger 7 can be turned only on the side where the cam face 24 is formed. As the plunger 7 is allowed to be folded only in one direction, the plunger 7 can be pushed more stably than in the case of the structure where the plunger is allowed to be folded in both directions.

The holder portion 45 has a structure such that it houses therein a compression coil spring 49, and a part of the base-end side of a cartridge-holding portion 51, which is biased towards the tip side by the compression coil spring 49.

The cartridge-holding portion 51 has a through hole formed at the center within which the axle of the plunger 7 is adapted to fit slidably. The cartridge-holding portion 51 is a sleeve-like member, with its external diameter formed in a stepwise manner. A small-diameter portion of the cartridge-holding portion 51 towards the base-end side is slidably fitted into a fitting hole 53 formed in the end face of the holder portion 45 towards the base-end side. A large-diameter portion of the holder portion 45 towards the tip side is adapted to abut directly against the end face of the flange portion 25 of the cartridge 9 so as to push the cartridge. In the cartridge-holding portion 51, there is formed a pusher receiving hole 55 through which the cartridge-holding portion 51 abuts against the end face of the pusher 37 on the base-end side, whereby the cartridge-holding portion 51 is prevented from becoming detached from the plunger 7. An intermediate-diameter portion formed in the middle of the cartridge-holding portion 51 functions as a seat for the compression coil spring 49.

The thus constructed knuckle 17, compression coil spring 49, and cartridge-holding portion 51 are assembled with the syringe body 3 and the plunger 7 as shown in Figs. 1 to 6, using the securing screws 19 such that they can be freely turned or can be slidable.

The above-described constituent members of the cartridge syringe 1 except for the needle-holding member 5, namely the syringe body 3, the plunger 7, and the members forming the one-touch loading mechanism including the knuckle 17 and the cartridge-holding portion 51, are made of a tenacious material such that they can withstand repeated use. Examples of such material include metal materials such as titanium, stainless steel, and aluminum, and alloys containing these materials as main components. In particular, titanium is preferable as it is superior in terms of chemical resistance, heat resistance, lightness, and strength, and so on. Of course, other metal materials or non-metal materials such as synthetic resins, with similar performance can be used.

Hereafter, the cartridge 9, which is loaded in the cartridge syringe 1 of the present invention and is a constituent element of the ophthalmologic syringe set of the present invention, will be described. As shown in Figs. 1 to 6, the cartridge 9 comprises a cylindrical cartridge body 57 and a small-diameter portion 31 formed by somewhat narrowing the portion of the cartridge body 57 towards the tip side. The cartridge 9 also comprises the aforementioned flange portion 25 towards the base-end side with locking faces 27. The cartridge 9 further comprises a piston stopper 39 by which the open face of the cartridge body 57 at the base-end side is closed, the piston stopper 39 functioning as a piston for pushing out an ophthalmologic therapeutic agent that is loaded in the cartridge 9. The material of the cartridge body 57 may be glass, polyolefin plastics such as polyethylene or polypropylene, or plastics such as polyethylene terephthalate or polycarbonate, which provides superior visibility. Glass is preferable from the viewpoint of the stability of the contents. Indicated by the numeral 59 is a rubber stopper by which a small opening formed at the center of the small-diameter portion 31 is blocked. The use of the cartridge 9 with the small-diameter portion at the tip eliminates the need for the reversed needle in the prior art shown in Fig. 13. Thus, the structure of the device can be simplified, and the entry of broken rubber pieces into the drug can be prevented when the rubber stopper is stuck by the reverse needle.

The volume of the cartridge 9 is from 1.0 mL to 20.0 mL, and the volume of ophthalmologic therapeutic agent that can be loaded in the cartridge 9 is from 0.1 mL to 20.0 mL. When the loaded ophthalmologic therapeutic agent is an aqueous solution containing sodium hyaluronate as an active ingredient, the volume of the ophthalmologic therapeutic agent to be loaded should preferably be between 0.4 mL and 2.0 mL, in light of the amount required in surgery and the efficiency of loading the agent into the cartridge 9.

Examples of the ophthalmologic therapeutic agent used in the present invention include viscoelastic substances, perfusion solutions, mydriatics, miotics, antibiotics, antibacterial agents, anti-inflammatory agents, and local anesthetics. Examples of the viscoelastic substances include sodium hyaluronate, chondroitin sulfate, chitin, chitosan, collagen, cellulose derivatives, or an aqueous solution containing a mixture of the aforementioned components as an active ingredient. An example of a perfusion solution is an isotonic aqueous solution (containing monovalent and/or divalent ions, such as sodium ions, potassium ions, calcium ions, or magnesium ions, with a pH of 6.4 to 8.2 and optionally containing glutathione and glucose).

The mydriatics include epinephrine and drugs with similar effects (including derivatives thereof), and so on. The miotics include acetylcholine and drugs with similar effects (including derivatives thereof and cholinesterase inhibitors), and so on. The antibiotics include aminoglycoside and β-lactam antibiotics, and so on. The antibacterial agents include quinolone synthetic antibacterial agents, and so on. The anti-inflammatory agents include steroid and nonsteroidal anti-inflammatory agents, and so on. The local anesthetics include lidocaine and drugs with similar effects (including derivatives thereof).

These ophthalmologic therapeutic agents can be locally administered to an affected site as drugs for the treatment and/or prevention of ophthalmologic diseases, particularly cataracts, in mammals including humans, for example. They can also be used as ophthalmologic surgery-aiding substances when an intraocular lens is implanted, for example.

As the aforementioned salts of hyaluronic acid, sodium salt is normally used, although potassium salt may also be used. The hyaluronic acid or the salts thereof that may be used in the present invention should preferably have a molecular weight of the order of 500,000 to 8,000,000, and more preferably from 500,000 to 3,900,000.

The molecular weight should be between 500,000 and 1,200,000 if an endothelium protection effect is to be obtained. If the hyaluronic acid or salts thereof are to be used for retaining the anterior chamber, the molecular weight should preferably be in the range between 1,900,000 and 3,900,000. Further, some surgeons prefer molecular weight between 1,500,000 and 3,900,000 for reasons of operability during surgery. Thus, the molecular weight is varied to provide different features depending on the aforementioned purposes. It is noted here that the term "molecular weight" in the present specification refers to the viscosity average molecular weight.

The concentration of the aforementioned hyaluronic acid or salts thereof should preferably be between 200 and 5,000 µg/mL. If the concentration of hyaluronic acid or salts thereof is less than 200 µg/mL or more than 5,000 µg/mL, the extension-promoting effect of the corneal epithelium layer drops, and this is not preferable. In the case of ophthalmologic liquid preparations, a concentration of 5,000 µg/mL or more would result in difficulty in handling and administration (application), and is therefore not preferable.

In cases where the ophthalmologic therapeutic agent is an aqueous solution containing hyaluronic acid or salts thereof as an active ingredient, additives other than hyaluronic acid may be added appropriately. Such additives include salts such as sodium chloride, disodium hydrogenphosphate, potassium hydrogenphosphate and chondroitin sulfate, and anti-inflammatory agents such as glycyrrhizin.

When an ophthalmologic therapeutic agent using the hyaluronic acid or salts thereof used in the present invention is clinically applied as an ophthalmologic liquid preparation, sodium hyaluronate with a molecular weight of 500,000 to 8,000,000, preferably from 500,000 to 3,900,000, is dissolved in phosphate-buffered physiological saline to a concentration of 200 to 5,000 µg/mL, and 0.1 to 2 mL of the solution is administered per unit dose. The safety of hyaluronic acid (in terms of its toxicity and noninflammatory nature) has been confirmed through a number of experiments. For example, with regard to its ocular toxicity, the absence of pungency and antigenicity has been confirmed by an experiment with rabbits (N. Ueno, et al., Folia Ophthalmologica Japonica, 35: 584 (1984) and 35: 803 (1984)).

The cartridge syringe 1 of the present invention and the ophthalmologic syringe set equipped with the cartridge syringe have the above-described structures. In the following, the operation of these devices will be described. First, the handle 35 is pulled to bring the plunger 7 up to the extreme base-end side. In this state, the plunger 7 is folded to the side on which the cam faces 24 are formed, whereby the cam faces 24 slide on the cam slide faces 28, allowing the plunger 7 to be turned.

Meanwhile, the cartridge 9 is prepared separately from the cartridge syringe 1, and the cartridge 9 is inserted into the syringe body 3 through its open end towards the base-end side, thus the cartridge 9 is loaded into the cartridge-housing portion 11. The cartridge 9 is positioned such that the locking faces 27 formed on the flange portion 25 of the cartridge 9 engage the insides of the locking claws 23 formed on the syringe body 3.

The plunger 7 is then turned in the opposite direction to bring it back to the linear position with respect to the syringe body 3. As the plunger 7 is linearly positioned, the cartridge-holding portion 51 is moved to the tip side by the biasing force of the compression coil spring 49. As a result, the flange portion 25 of the cartridge 9 is held between the cartridge-holding portion 51 and the flange support portion 26. Thus, the cartridge 9 is secured relative to the syringe body 3.

The plunger 7 is then pushed in the axis direction or rotated in a predetermined direction about the axis so as to cause the push-in portion 41 on the pusher 37 to be inserted into the insertion hole 43 formed in the piston stopper 39 in the cartridge 9. Thus, the piston stopper 39 and the plunger 7 are coupled such that they can be moved as an integral component. Thereafter, the rubber stopper 59 fitted at the tip of the cartridge 9 is removed, and the needle holding member 5 is attached to the small-diameter portion 31 of the cartridge 9 through the tip of the syringe body 3. In this state, the cartridge syringe 1 is ready for use, as shown in Figs. 5(a) and 6(a).

When using the cartridge syringe 1, the user rests his or her index and middle fingers, for example, on the gripping portion 15 of the syringe body 3, places his or her thumb on the handle 35 of the plunger 7, and then pushes the plunger 7 towards the tip side. As the plunger 7 is pushed, the piston stopper 39 fixed to the plunger 37 via the push-in portion 41 and insertion hole 43 is also moved towards the tip side. Consequently, the ophthalmologic therapeutic agent which is filled into the cartridge body 57 is pushed out via the needle 29, and is administered.

In the second embodiment of the ophthalmologic syringe set of the invention shown in Figs. 2, 4 and 6, as the plunger 7 is pulled after administration of the ophthalmologic therapeutic agent, the push-in portion 41 can be easily detached from the insertion hole 43 of the piston stopper 39 because of the tapered shape of the push-in portion 41. Thus, the plunger 7 can be pulled back to the extreme base-end position, and can be then easily folded again in the same manner as described above. Then, a cartridge 9 can be removed after use and a new cartridge 9 can be immediately loaded.

When the plunger 7 is folded, the plunger 7 is pulled to the extreme base-end position because when the plunger 7 is pushed to some extent, the side face of the pusher 37 abuts the internal surface of the syringe body 3 or cartridge body 57 such that the turning of the plunger 7 is structurally prevented.

It is noted that in this structure, the plunger 7 can be prevented from being accidentally folded against the will of the user during use, which could result in the cartridge 9 becoming detached.

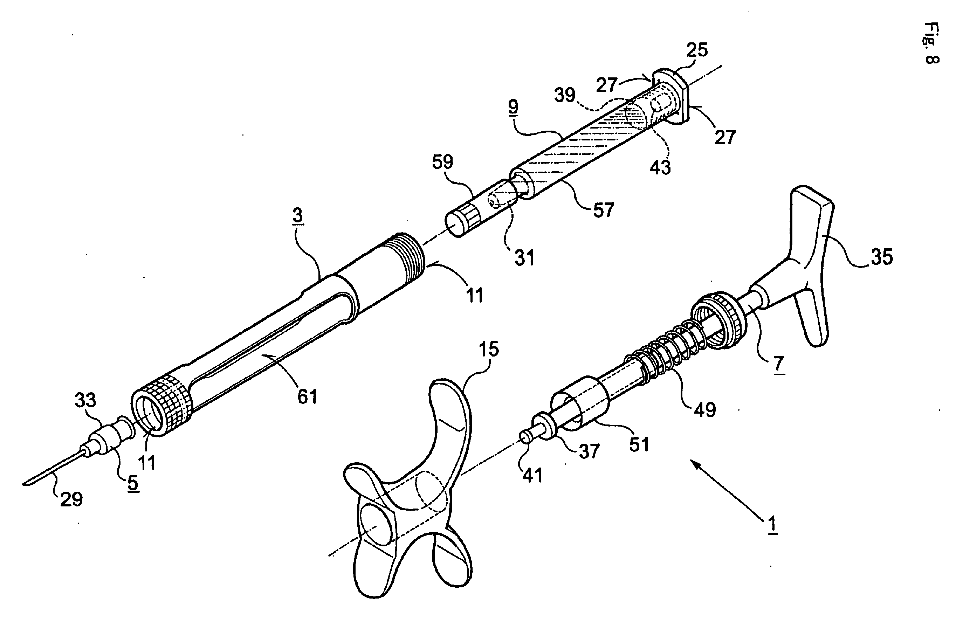

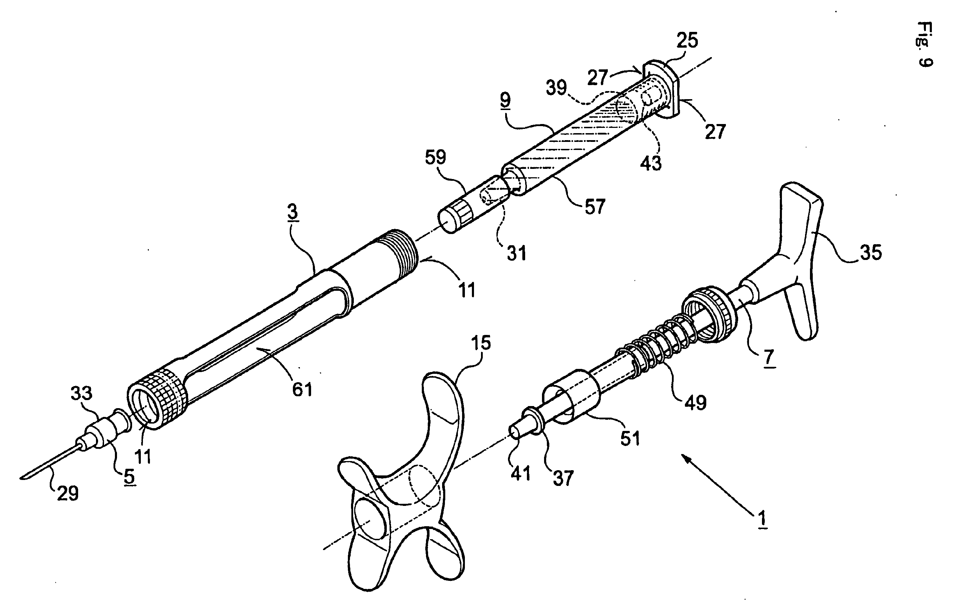

Another embodiment of the invention will be described. Figs. 8 and 9 show the embodiment in which the cartridge 9 is loaded into the cartridge-housing portion 11 through a relatively large receiving opening portion 61 formed in the syringe body 3, basically without eliminating the coupling between the syringe body 3 and the plunger 7. Also in this structure, the cartridge 9 can be housed in the cartridge-housing portion 11 by a simple operation, and this structure is included in the one-touch loading mechanism described in the present specification.

While in the embodiment shown in Figs. 8 and 9 the gripping portion 15 is formed separately from the syringe body 3, the gripping portion 15 may be secured to the syringe body 3, as in the embodiment of Figs. 1 to 6.

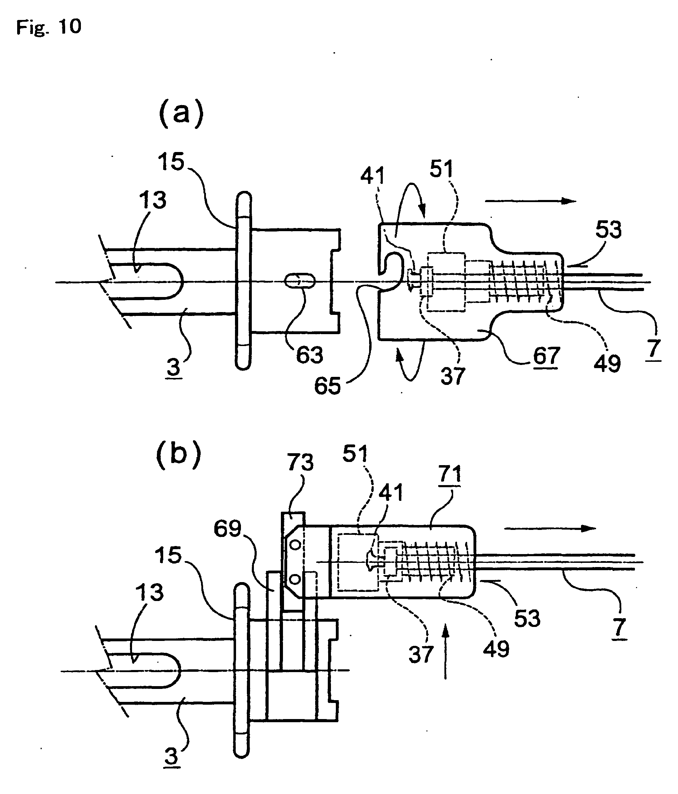

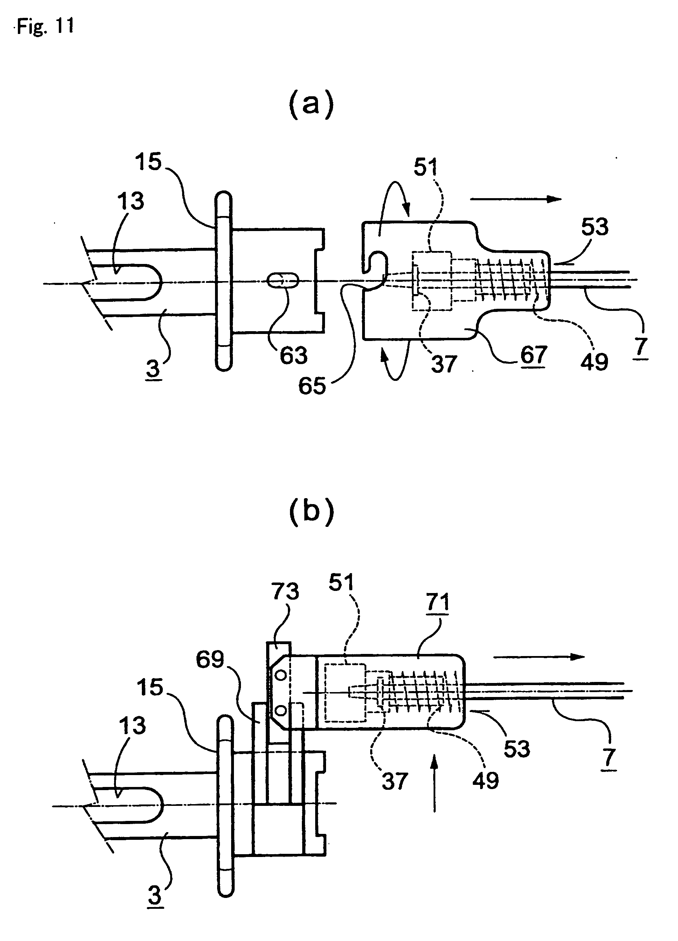

However, in the embodiment of Figs. 8 and 9, the length of the syringe body 3 tends to become larger for structural reasons. This disadvantage is overcome in the embodiment shown in Figs. 1 to 6 and the embodiment shown in Figs. 10 and 11, the latter of which is described below.

Figs. 10 and 11 show further other embodiments of the one-touch loading mechanism. The embodiment shown in Figs. 10(a) and 11 (a) is of a dismantling type, in which the syringe body 3 and the plunger 7 can be detached by a simple operation. Specifically, a locking claw 63 is provided at the base-end portion of the syringe body 3, and a locking groove 65 that is adapted to engage the locking claw 63 is provided in a plunger holder 67. Thus, the plunger 7 can be detached from the syringe body 3 by turning the plunger holder 67 slightly.

The embodiment shown in Figs. 10(b) and 11(b) is of a sliding type in which the plunger 7 can be slid in a direction perpendicular to the axis of the syringe body 3 by a simple operation. Specifically, a guide rail 69 is provided on the syringe body 3, and a slider 73 adapted to engage the guide rail 69 is provided on a plunger holder 71 by which the plunger 7 is retained. Thus, the plunger 7 can be slid to the side of the syringe body 3.

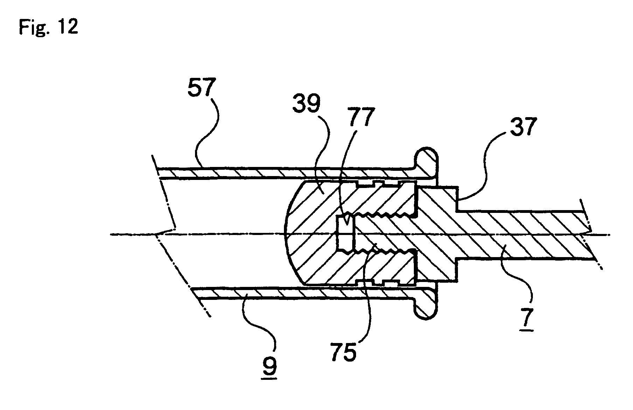

Fig. 12 shows yet another embodiment of an integral coupling type. In this embodiment, an externally threaded convex portion 75 is formed on the pusher 37, while an internally threaded concave portion 77 is formed in the piston stopper 39. In this structure, the plunger 7 can be more strongly coupled with the piston stopper 39 by threading the plunger 7 into the piston stopper 39.

### INDUSTRIAL APPLICABILITY

In accordance with the invention recited in claims 1 to 11, the cartridge syringe can be used repeatedly. Thus, the waste is only the cartridge, so that the amount of waste can be reduced considerably as compared with the case of conventional disposable syringes. The loading of the cartridge into the cartridge syringe can be performed in a short time by a simple operation, using the one-touch loading mechanism. Further, as it is only the cartridge that must be contained hermetically in a blister package for preventing contamination with microbes or the like, the blister package and the outer package can be reduced in size. Consequently, the problem of storage space in drug storage refrigerators can be overcome. Thus, in order to reduce the amount of waste and the necessary storage space, it is indispensable to supply only a cartridge into which the ophthalmologic therapeutic agent has been loaded.

It has been confirmed that, in accordance with the present invention, the volume of the blister package and the outer package as well as the weight of waste products can be effectively reduced as compared with the prior art.

In accordance with the invention recited in claim 3, the cartridge can be firmly held in the cartridge-housing portion, so that there is no danger of the cartridge becoming detached from the cartridge-housing portion.

In accordance with the invention recited in claim 4, the cartridge can be easily loaded and unloaded by the folding of the plunger.

In accordance with the invention recited in claim 5, the plunger 7 can be pushed more stably than in a case where the plunger can be folded in both directions.

In accordance with the invention recited in claim 6, the loading depth of the cartridge can be reliably controlled when the cartridge is loaded into the cartridge-housing portion.

In accordance with the invention recited in claim 7, the rotation of the cartridge around the longitudinal axis can be prevented, so that the movement of the cartridge can be prevented when the plunger is pushed. Thus, the drug can be stably injected.

In accordance with the invention recited in claim 8, the flange of the cartridge is held between the cartridge-holding portion and the flange support portion, so that the cartridge can be prevented from wobbling in the cartridge-housing portion.

In accordance with the inventions recited in claims 11, 12, and 14, the movement of the plunger is directly translated into the movement of the piston stopper, so that the operability of the plunger and the responsiveness of the piston stopper can be improved. Thus, these inventions can handle the administration of a minute amount of ophthalmologic therapeutic agent.

In accordance with the invention recited in claim 13, the push-in portion of the plunger is tapered such that the diameter decreases towards the tip of the push-in portion. Thus, the push-in portion can be stably inserted into the insertion hole in the piston stopper when the plunger is pushed, while the push-in portion can be easily pulled out of the insertion hole of the piston stopper during the pulling operation of the plunger. Accordingly, the plunger can be easily pulled back to the extreme base-end position, where the plunger 7 can be easily folded again.

In accordance with the invention recited in claim 16, the aforementioned advantageous effects can be realized in an ophthalmologic syringe set.

In accordance with claims 17 to 19, the invention can be widely applied to the treatment or prevention of ophthalmologic diseases, such as cataracts, as a result of its efficacy and safety.

In accordance with the invention recited in claim 20, an appropriate cartridge can be selected that is suitable for the dose of an ophthalmologic therapeutic agent used for surgery, so that an appropriate dose of the ophthalmologic therapeutic agent can be administered in a single operation.

## Claims

1. A cartridge syringe for use with a flanged cartridge that is filled with an ophthalmologic therapeutic agent, wherein the cartridge syringe comprises a syringe body having a cartridge-housing portion, a needle holding member that is disposed in a connectable manner to the tip of the syringe body, and a plunger that is slidably disposed on a base-end side of the syringe body, the plunger having a pusher at the tip thereof, wherein a one-touch loading mechanism is provided in the syringe body or at a coupling portion between the syringe body and the plunger, the one-touch loading mechanism allowing the position of the plunger to be temporarily changed such that the cartridge can be loaded into the syringe body.

2. The cartridge syringe of claim1 wherein the one-touch loading mechanism comprises a knuckle by which the cartridge syringe body and the plunger are coupled in a foldable manner, whereby the cartridge can be loaded by folding the plunger.

3. The cartridge syringe of claim 1 or 2, wherein a cartridge-holding portion is mounted around the plunger slidably with respect to the syringe body, the cartridge-holding portion being biased towards the tip of the cartridge syringe such that when the cartridge is loaded in the cartridge-housing portion, the cartridge can be held in place in such a way that the base-end side of the cartridge is pushed by the cartridge-holding portion.

4. The cartridge syringe of claim 2 or 3, wherein a cam face is formed at the tip of the knuckle such that the cam face can slide on a cam slide face formed at the base-end side of the syringe body, thereby allowing the knuckle to be turned, and wherein, when the plunger is fully pulled and then folded towards the side on which the cam face is formed, the cam face slides on the cam slide face, thereby allowing the folding of the plunger.

5. The cartridge syringe of claim 4 wherein the plunger is adapted to be foldable in only a single direction.

6. The cartridge syringe of any of claims 1 to 5 wherein a flange support portion is formed on the base-end side of the syringe body such that, when the cartridge is loaded into the cartridge-housing portion, the flange portion of the cartridge abuts the flange support portion, thereby limiting the insertion of the cartridge.

7. The cartridge syringe of any of claims 1 to 6 wherein two locking claws are formed on the base-end side of the syringe body, the locking claws opposed to each other and extending in the longitudinal direction, and wherein the loading of the cartridge is completed when two locking faces formed on the flange portion of the cartridge for regulating the orientation of the cartridge are positioned such that they are opposite the insides of the locking claws, and when the loading of the cartridge is thus completed, the locking faces on the cartridge engage the inside of the locking claws, so that the free rotation of the cartridge around the longitudinal axis of the cartridge is limited.

8. The cartridge syringe of any of claims 3 to 7 wherein, when the cartridge is loaded in the cartridge-housing portion, as the plunger is turned to be linearly oriented with respect to the syringe body, the cartridge-holding portion is biased towards the syringe body such that the flange portion of the cartridge is retained between the cartridge-holding portion and the flange support portion.

9. The cartridge syringe of claim 1 wherein the one-touch loading mechanism is structured such that it can be dismantled by slightly turning the plunger holder holding the plunger around the longitudinal axis of the syringe body.

10. The cartridge syringe of claim 1 wherein the one-touch loading mechanism is structured such that the plunger holder holding the plunger is slid in a direction perpendicular to the longitudinal axis of the syringe body.

11. The cartridge syringe of any of claims 1 to 10 wherein an integral coupling structure is provided between a pusher provided on the tip of the plunger and a piston stopper provided on the base-end side of the cartridge, whereby the pusher and the piston stopper are coupled in an integral manner.

12. The cartridge syringe of claim 11 wherein the integral coupling structure comprises a push-in portion provided on the pusher and an insertion hole formed in the piston stopper.

13. The cartridge syringe of any of claims 1 to 12 wherein a tapered push-in portion is formed more towards the tip side than the pusher, the push-in portion being adapted to be inserted into an insertion hole formed on the base-end side of the piston stopper of the cartridge.

14. The cartridge syringe of claim 11 wherein the integral coupling structure comprises an externally threaded convex portion formed on the pusher and an internally threaded concave portion formed on the piston stopper.

15. A cartridge filled with an ophthalmologic therapeutic agent for use in combination with the cartridge syringe of any of claims 1 to 14.

16. An ophthalmologic syringe set which comprises the cartridge syringes of any of claim 1 to 14 and a cartridge filled with an ophthalmologic therapeutic agent.

17. The ophthalmologic syringe set of claim 16 wherein the ophthalmologic therapeutic agent with which the cartridge is filled is a viscoelastic substance.

18. The ophthalmologic syringe set of claim 16 wherein the ophthalmologic therapeutic agent with which the cartridge is filled is an aqueous solution containing hyaluronic acid or its salts as an active ingredient.

19. The ophthalmologic syringe set of claim 16 wherein the ophthalmologic therapeutic agent with which the cartridge is filled is an aqueous solution containing sodium hyaluronate as an active ingredient.

20. The ophthalmologic syringe set of any of claims 16 to 19 wherein the volume of the cartridge is from 1.0 mL to 20.0 mL.
